Europäisches Patentamt

European Patent Office

Office européen des brevets

(11) Veröffentlichungsnummer: **0 175 990 B1**

(12) **EUROPÄISCHE PATENTSCHRIFT**

(45) Veröffentlichungstag der Patentschrift: **19.06.91**

(51) Int. Cl.⁵: **G01N 33/52**, //G01N33/545, G01N33/546,C12Q1/00

(21) Anmeldenummer: **85111366.2**

(22) Anmeldetag: **09.09.85**

(54) **Membran für Reagenzträgerschichten, Verfahren zu deren Herstellung, sowie deren Verwendung in analytischen Mitteln und Analysenverfahren.**

(30) Priorität: **22.09.84 DE 3434822**

(43) Veröffentlichungstag der Anmeldung:
**02.04.86 Patentblatt 86/14**

(45) Bekanntmachung des Hinweises auf die
Patenterteilung:
**19.06.91 Patentblatt 91/25**

(84) Benannte Vertragsstaaten:
**AT BE CH DE FR GB IT LI LU NL SE**

(56) Entgegenhaltungen:
**EP-A- 0 016 387**
**WO-A-79/01081**
**DE-A- 2 739 008**
**US-A- 3 993 451**
**US-A- 4 356 149**

(73) Patentinhaber: **MILES INC.**
**1127 Myrtle Street**
**Elkhart,IN 46515(US)**

(72) Erfinder: **Piejko, Karl-Erwin, Dr.**
**Roggendorfstr. 57**
**W-5000 Köln 80(DE)**
Erfinder: **Bömer, Bruno, Dr.**
**Max Planck-Str. 33**
**W-5060 Bergisch-Gladbach 2(DE)**
Erfinder: **Bartl, Herbert, Dr.**
**Eichendorffweg 10**
**W-5068 Odenthal(DE)**
Erfinder: **Frank, Georg, Dr., c/o Ames QSD**
**Miles Laboraties Inc., P.O.Box 70**
**Elkhart, IN 46514(US)**

(74) Vertreter: **Dänner, Klaus, Dr. et al**
**c/o Bayer AG Konzernverwaltung RP Patente**
**Konzern**
**W-5090 Leverkusen 1 Bayerwerk(DE)**

EP 0 175 990 B1

## Beschreibung

Die Erfindung betrifft neuartige Membranen und ihre Verwendung als Reagenzträgerschichten für analytische Mittel, die zur trockenchemischen Bestimmung von Bestandteilen wäßriger Lösungen, vornehmlich Körperflüssigkeiten wie Blut, Urin, Serum, Milch oder dergleichen eingesetzt werden können, sowie deren Herstellung aus einer Wasser-in-Öl-Emulsion eines hydrophilen, Wasser enthaltenden und vorzugsweise wasserlöslichen Polymers und der Lösung eines hydrophoben, in Wasser unlöslichen, filmbildenden Polymers.

Trockenchemische Methoden bieten gegenüber wäßrigen Analysemethoden eine Reihe von Vorteilen, die zu einer starken Verbreitung der Anwendung von Teststreifen vor allem im diagnostischen Bereich geführt haben. Vorteile trocken arbeitender Analysensysteme liegen z.B. in der einfachen Handhabung, so daß auch ungeschulte Personen damit umgehen können, in der kurzen Analysenzeit sowie der Tatsache, daß keine flüssigen Reagenzien benötigt werden und somit auch keine Entsorgungsprobleme von Chemikalien auftreten.

Zur Ausführung einer Analyse mittels Teststreifen werden diese in die zu untersuchende Flüssigkeit wie z.B. Urin eingetaucht, herausgezogen und nach einer bestimmten Zeit die auftretende Färbung entweder durch Vergleich mit einer Farbskala halbquantitativ beurteilt oder die Intensität der Färbung wird mittels eines optischen Gerätes quantitativ gemessen, wodurch Rückschlüsse auf die Konzentration des zu analysierenden Bestandteils der wäßrigen Flüssigkeit in halbquantitativer bzw. quantitativer Form möglich sind. Ein häufig analysierter Bestandteil von Körperflüssigkeiten wie Blut oder Urin ist z.B. Glucose.

Besteht die zu untersuchende Flüssigkeit aus Vollblut, so müssen die Erythrozyten des Blutes von der Reagenzzone des Teststreifens ferngehalten oder nach einer gewissen Einwirkzeit der Blutprobe vom Teststreifen entfernt werden, da sonst die bei der Nachweisreaktion für die zu bestimmende Komponente des Blutes entstandene Färbung nicht beurteilt oder gemessen werden kann.

Häufig verwendetes Material für Teststreifen ist Papier, das mit Reagenzlösungen getränkt ist und direkt oder in Verbindung mit polymeren Trägermaterialien oder zusätzlichen Komponenten wie hydrophoben Polymeren oder semipermeablen Membranen eingesetzt wird. Solche Testmittel sind z.B. in den US-PS 3 092 463, 3 092 465 und der EP-A 78 971 beschrieben. Faserige Materialien wie Papier zeigen allerdings gravierende Nachteile als Reagenzträgerschichten bei Teststreifen. Diese Nachteile sind in der US-PS 4 042 335 ausführlich beschrieben. Hier sollen nur die schlecht reproduzierbaren Analysenwerte angesprochen werden, die auf der Uneinheitlichkeit des Papiermaterials beruhen und allenfalls eine halbquantitative Interpretation der Ergebnisse zulassen.

Quantitativ auswertbare Analysenergebnisse werden mittels Teststreifen mit synthetischen Reagenzträgerschichten erhalten, die in einheitlicher Form hergestellt werden können. Solche Reagenzträgerschichten bestehen z.B. gemäß US-PS 3 992 158 und DE-OS 3 222 366 aus hydrophilen Polymeren die Gelatine oder Polyvinylalkohol. Reagenzträgerschichten dieser Art können allerdings nur in Kombination mit anderen zusätzlichen Schichten eingesetzt werden, wie sie in den oben genannten Schriften beschrieben werden. Die zusätzlichen Schichten übernehmen Hilfsfunktionen, die von der hydrophilen Reagenzträgerschicht alleine nicht erfüllt werden können. Als solche zusätzliche Hilfsschichten seien z.B. Spreitschichten genannt, die eine flüssige Probe verteilen oder spreiten, so daß auf eine exakte Probendosierung verzichtet werden kann, oder Reflexionsschichten, die Licht reflektieren, das durch die transparente Trägerfolie und die Reaktionsschicht fällt, und so eine Messung der Farbintensität von der Trägerseite her ohne Störung durch trübe oder gefärbte Anteile der untersuchten Flüssigkeit ermöglichen. Die trüben oder gefärbten Anteile wie z.B. die Erythrozyten im Blut müssen natürlich am Durchtreten durch die Reflexionsschicht gehindert werden, wozu u.U. eine weitere Schicht als Blockierschicht eingesetzt werden muß.

Solche Mehrfachschichten-Analysensysteme sind naturgemäß schwierig herzustellen, da eine Reihe ganz unterschiedlicher Schichten in engen Kontakt miteinander gebracht werden müssen, z.B. durch Aufkleben oder Auflaminieren oder durch Übereinandergießen flüssiger Gießmischungen. Aufgrund der unterschiedlichen Hydrophilie bzw. Hydrophobie der einzelnen Schichten kann häufig eine schlechte Haftung beobachtet werden. Weitere Nachteile von Mehrschichtensystemen sind ausführlich z.B. in der US-PS 4 356 149 beschrieben.

Die Reagenzträgerschichten aus Gelatine oder Polyvinylalkohol in solchen Mehrfachschicht-Testmitteln weisen selbst eine Reihe von Nachteilen auf: Sie behindern u.a. die Diffusion von Reaktionskomponenten, worauf z.B. in der DE-OS 3 222 366 hingewiesen wird und erhöhen damit den Zeitbedarf für die Durchführung einer Analyse. Weiterhin diffundieren vor allem hydrophobe und hochmolekulare Stoffe nicht oder nur sehr langsam und können deshalb auch nicht quantitativ nachgewiesen werden, wie in der DE-OS 3 235 658 aufgezeigt wird.

Auch Reagenzträgerschichten aus hydrophoben Polymeren wie Ethylcellulose, Acetylcellulose oder

EP 0 175 990 B1

Polyisobutylen, die gemäß US-PS 3 630 957 verwendet werden, führen aufgrund sehr kleiner Poren zu langen Reaktionszeiten (beschrieben in DE-PS 2 950 501) und lassen den Nachweis hochmolekularer Stoffe nicht zu.

Höhere Reaktionsgeschwindigkeiten und damit kürzere Analysenzeiten werden mit porösen Reagenzträgerschichten erzielt. Die Poren saugen mittels Kapillarkräften einen Teil oder die ganze aufgebrachte Flüssigkeit auf und verteilen sie gleichmäßig innerhalb der Reagenzträgerschicht, so daß die wäßrigen Bestandteile schnell in Kontakt mit den in der Reagenzträgerschicht verteilten Reagenzien kommen. Es ist technisch möglich, die Poren so einzustellen, daß einerseits hochmolekulare Bestandteile der wäßrigen Probe noch eindringen können, andererseits aber störende, größere Bestandteile wie z.B. die Erythrozyten des Blutes auf der Oberfläche der Schicht verbleiben und entfernt werden können.

Eine solche poröse oder auch saugende Reagenzschicht ist in der DE-OS 2 910 134 beschrieben. Ein wasserfester Film aus Polyvinylpropionat wird durch Zugabe fester, unlöslicher Partikel - Filmöffner genannt - aus z.B. Titandioxid oder Cellulose porös gemacht und kann dann als Reagenzträgerschicht zum Nachweis hochmolekularer Bestandteile wäßriger Systeme dienen. Das Erzielen poröser Strukturen durch Zugabe von Pigmenten zu Polymerlatices ist schon lange bekannt und z.B. in "Progress in Org. Coatings 11, 267-285 (1983)" referiert. Nachteilig bei Reagenzschichten dieser Art ist die Tatsache, daß die Natur des Filmöffners keinen Einfluß auf den Verlauf der Nachweisreaktion hat, die in den Poren bzw. an der Grenzfläche zum hydrophoben Polymer abläuft, d.h. es bestehen wenig Variationsmöglichkeiten, um die Reagenzschicht für bestimmte Nachweissysteme zu optimieren.

Die geringe Variationsbreite gilt auch für das filmbildende Polymer, als das in den Beispielen der DE-OS 2 910 134 ausschließlich Polyvinylpropionat verwendet wird.

Ein anderes poröses analytisches Mittel ist in EP-A 13 156 beschrieben. Es wird durch Verkleben unlöslicher Teilchen mit einem unlöslichen Kleber hergestellt, wobei definierte Hohlräume gebildet werden, die sich vor allem für den Transport großer Moleküle wie Vollblut eignen sollen. Dieses System besitzt viele Nachteile, die in der DE-OS 3 235 658 ausführlich beschrieben sind. Besonders nachteilig ist dabei die Tatsache, daß die dreidimensionale Struktur nur aus hydrophoben Bestandteilen zusammengesetzt ist, und deshalb wäßrige Flüssigkeitsproben schlecht festgehalten werden.

Hydrophile, unlösliche Partikel, die farbbildende Reagenzien enthalten und auf Papier geklebt sind, werden gemäß US-PS 3 993 451 als Reagenzträger verwendet. Die Größe der Partikel ist wegen der Pulverform der eingesetzten Materialien schlecht kontrollierbar und der Farbtest daher irrtumsanfällig. Gleichmäßige, hydrophile, aber wasserunlösliche Perlen mit Mikrovakuolen werden in der DE-PS 2 950 501 als Reagenzträger beansprucht. Das Verfahren zu ihrer Herstellung ist jedoch äußerst kompliziert, da das Polymer halbdurchlässig sein muß, was nur unter ganz bestimmten Herstellungsbedingungen erreicht wird. Die Mikrovakuolen werden durch Dispergieren einer wäßrigen Reagenzlösung in einer Lösung von Cellulosetriacetat in Methylenchlorid erzeugt. Die dabei entstandene Wasser-in-Öl-Dispersion wird zur Herstellung der Perlen ihrerseits in einer 2. Wasserphase emulgiert, wobei eine Wasser-in-Öl-in-Wasser-Emulsion entsteht. Aus dieser werden die wasserunlöslichen Perlen durch Verdampfen des organischen Lösungsmittels und anschließende Filtration isoliert, getrocknet und durch Sieben mit Drahtsieben nach der Größe fraktioniert. Perlen der gewünschten Größe (<100 $\mu$m) werden dann als Mono- oder Doppelschicht auf eine Trägerfolie aufgeklebt, wodurch erst der fertige Teststreifen erhalten wird. Ein Vorteil dieser Reagenzträgerschichten liegt im Prinzip darin, daß hydrophile und hydrophobe Reagenzien gut miteinander kombiniert werden können, da in dem Dreiphasensystem während der Herstellung der Perlen sowohl eine organische als auch eine wäßrige Phase vorhanden sind. Voraussetzung ist allerdings, daß die Bildung des störanfälligen Dreiphasensystems durch die Reagenzien selbst nicht beeinflußt wird und das gebildete Polymer halbdurchlässig bleibt.

Eine einfache Kombination hydrophober Reagenzien mit einer hydrophilen Binderschicht wird gemäß US-PS 4 089 747 und 4 356 149 dadurch erreicht, daß hydrophobe Reagenzien als Lösung in einem organischen Lösungsmittel oder als feine, hydrophobe Partikel, eingeschlossen in einem hydrophilen Binder, verteilt werden. Diese Reagenzschichten sind Bestandteil von Mehrfachschicht-Analysensystemen, die die bereits diskutierten Nachteile aufweisen.

Eine andere Analyseneinheit, die hydrophile und hydrophobe Bestandteile enthält, besteht nach der DE-OS 3 235 658 u.a. aus einem Kern-Hülle-Polymer mit hydrophobem Kern und hydrophiler Hülle. Eine solche Analyseneinheit zeigt ein besseres Festhaltevermögen für hydrophobe oder hochmolekulare Substanzen. Da die einzelnen Teilchen durch Adhäsion der hydrophilen Außenschichten miteinander verbunden sind, die Adhäsion aber durch Einwirken einer wäßrigen Probe vermindert wird, sind derartige Schichten nicht wischfest. Für die Analyse von Vollblut muß deshalb eine zusätzliche Barriereschicht zum Zurückhalten der Erythrozyten auf die eigentliche Reagenzschicht aufgebracht werden, d.h. ein Mehrschichtensystem mit seinen bekannten Nachteilen aufgebaut werden.

3

Ebenfalls Bestandteil von Mehrschichtensystemen sind in der US-PS 4 403 408 beschriebene Schichten, die entweder ein hydrophobes Lösungsmittel, dispergiert in einer hydrophilen Phase, enthalten oder eine wäßrige Phase, dispergiert in einer hydrophoben Phase. Die dispergierte Phase hat die Aufgabe, im ersteren Fall hydrophobe, im letzteren Fall hydrophile Komponenten der zu untersuchenden Probe herauszufiltern, während die restliche Lösung ungehindert hindurchtreten kann. Die dispergierte wäßrige Phase enthält z.B. komplexierende Reagenzien wie quaternäre Alkylammonium- oder Phosphoniumsalze, die in Form von Seitengruppen auch Bestandteil von Polymeren sein können. Die Filterwirkung der zweiphasigen Schichten soll das Zusammentreffen hydrophiler und hydrophober Reagenzien bzw. Probenbestandteile verhindern, was eine Verwendung als alleinige Reagenzträgerschicht ausschließt.

EP-A 78 040 beschreibt einen Film, der aus einem organischen, hydrophoben Polymer und hydrophilen Anteilen, nämlich Proteinen wie Collagen oder Oligopeptiden besteht und als Wundverschluß dienen kann. Der Film ist zwar dampf- nicht jedoch flüssigkeitsdurchlässig und deshalb als Reagenzträgerschicht für die Analyse wäßriger Flüssigkeiten nicht geeignet.

Aus dem vorbeschriebenen Stand der Technik wird insgesamt ersichtlich, welche Anforderungen ein analytisches Mittel zum Bestimmen von Bestandteilen wäßriger Flüssigkeiten erfüllen sollte. Diese Anforderungen seien zur besseren Übersicht hier kurz zusammengefaßt:

1. Einfache, reproduzierbare Herstellung des Testmittels.

2. Alle Funktionen üblicher Testsysteme können in einer Reagenzschicht vereinigt werden, wobei besonders die Fähigkeit zum Spreiten einer wäßrigen Probe, ausreichende Wischfestigkeit zum Abwischen der Erythrozyten bei Verwendung von Vollblut als Analysenflüssigkeit sowie ein schnelles Eindringen der flüssigen Probe zu erwähnen sind.

3. Der Nachweis hochmolekularer und hydrophober Substanzen kann durch geeignete Modifizierung der Reagenzträgerschicht ermöglicht werden.

4. Hydrophile und hydrophobe Reagenzien können einfach in einem Nachweissystem kombiniert werden.

5. Das System bietet breite Variationsmöglichkeiten für eine Optimierung auf spezielle analytische Probleme.

Keines der vielen bereits bekannten analytischen Mittel erfüllt alle diese Anforderungen hinreichend. Es war daher Aufgabe der vorliegenden Erfindung, ein Analysensystem bereitzustellen, das diese fünf Anforderungen weitgehend erfüllt.

Die Aufgabe konnte dadurch gelöst werden, daß in einer Reagenzträgerschicht eines oder mehrere filmbildende, wasserunlösliche, organische Polymere mit einem oder mehreren wasserenthaltenden, organischen Polymeren und Nachweisreagenzien kombiniert werden.

Gegenstand der Erfindung ist eine wasser aufnehmende Membran für Reagenzträgerschichten in analytischen Mitteln zum trockenchemischen Nachweis von Bestandteilen wäßriger Probelösungen, dadurch gekennzeichnet, daß die Membran ein oder mehrere hydrophobe filmbildende, wasserunlösliche organische Polymere aus der Gruppe Polykohlenwasserstoffe, Polydiene, Polyvinylverbindungen, Polyacrylate bzw. -methacrylate, Polycarbonate, Polyacetale, Polyether, Polyurethane, Phenolharze, Polyester, Polysulfone, Polyamide bzw. -imide und organolösliche Polysaccharide enthält, in welchen ein oder mehrere hydrophile organische Polymere, die mindestens 20 Gewichtsprozent Wasser aufnehmen als diskrete Partikel verteilt sind. Vorzugsweise sind die hydrophilen Polymere in den Poren der Membran eingelagert, deren kohärente Phase aus den filmbildenden, wasserunlöslichen Polymeren besteht.

Die erfindungsgemäßen Membranen sind erhältlich, indem man die Lösung eines oder mehrerer filmbildender, wasserunlöslicher organischer Polymerer mit einer Dispersion eines oder mehrerer hydrophiler, wasserenthaltender Polymere in einem organischen Lösungsmittel mischt, die Mischung auf eine Unterlage aufbringt und trocknet.

Bei einer anderen Methode zur Herstellung der erfindungsgemäßen Membranen wird das hydrophile, wasserenthaltende Polymer in einer Lösung des filmbildenden, wasserunlöslichen Polymers in einem organischen Lösungsmittel dispergiert, diese Dispersion, die in ihrer Zusammensetzung der obigen Mischung entspricht, auf eine Unterlage aufgebracht und getrocknet.

Als hydrophile organische Polymere sollen hier solche Polymere verstanden werden, die mindestens 20 Gew.-% Wasser aufnehmen. Bevorzugte Polymere nehmen 50 Gew.-% Wasser, besonders bevorzugte mehr als 100 Gew.-% Wasser auf. Am besten geeignet sind wasserlösliche Polymere, die zu mehr als 1 Gew.-% bei Raumtemperatur in Wasser löslich sind. Solche Polymere werden bevorzugt in Form einer wäßrigen Lösung im organischen Lösungsmittel dispergiert.

Erfindungsgemäße Reagenzträgerschichten bestehen aus einer der oben definierten Membranen, welche eines oder mehrere (vorzugsweise alle erforderlichen) Nachweisreagenzien für die Bestimmung eines Bestandteils der Probe enthält.

Erfindungsgemäße analytische Mittel zum trockenchemischen Nachweis von Bestandteilen wäßriger Probelösungen sind dadurch gekennzeichnet, daß sie mindestens eine der erfindungsgemäßen Reagenzträgerschichten aufweisen.

Im erfindungsgemäßen Analyseverfahren wird in an sich bekannter Weise das erfindungsgemäße Mittel mit der wäßrigen Probe in Kontakt gebracht und die auftretende Nachweisreaktion (z.B. Farbbildung, Fluoreszenz oder Lumineszenz) verfolgt.

Bei der Herstellung der erfindungsgemäßen Reagenzträgerschichten werden die Nachweisreagenzien entweder in der Mischung, aus der die Membran hergestellt wird, aufgelöst oder in Form einer Wasser-in-Öl-(W/O)-Dispersion hinzugefügt oder (weniger bevorzugt) durch nachträgliches Imprägnieren der Membran in dieser verteilt. Die dabei gegebenenfalls verwendeten organischen Lösungsmittel sollen die weiter unten angegebenen Kriterien erfüllen.

Die vorzugsweise feste Unterlage bei der Membranherstellung kann aus Glas, Metall oder einem synthetischen oder natürlichen Polymer bestehen, das beim Aufbringen und Trocknen der Mischung zur Herstellung der Membran bzw. Reagenzträgerschicht nicht vollständig aufgelöst oder nicht soweit angequollen wird, daß es seine äußere Form verändert. Die Unterlage kann transparent oder opak sein. Sie besteht vorzugsweise aus Polyethylenterephthalat, dem Pigmente zugesetzt sein können, um es lichtundurchlässig zu machen oder aus polyethylenbeschichtetem Papier.

Die Membran bzw. die Reagenzträgerschicht kann auch auf einer der oben genannten Unterlagen hergestellt, von dieser abgelöst und mittels eines geeigneten Klebers auf einen anderen Träger aufgebracht werden, der dann auch aus einem in üblichen organischen Lösungsmitteln löslichen Material wie z.B. Polycarbonat, Celluloseacetat oder, Polystyrol bestehen kann.

Als filmbildende, wasserunlösliche organische Polymere sind solche geeignet, die weniger als 1 Gew.-% Wasser durch Quellung aufnehmen, die sich zu mehr als 1 Gew.-% in einem der weiter unten beschriebenen organischen Lösungsmittel lösen und nach Verdampfen des Lösungsmittels einen Film ergeben. Die Polymere sind wasser lösliche organische Polymere aus der Gruppe Polykohlenwasserstoffe, Polydiene, Polyvinylverbindungen, Polyacrylate bzw. -methacrylate, Polycarbonate, Polyacetale, Polyether, Polyurethane, Phenolharze, Polyester, Polysulfone, Polyamide bzw. -imide und organolösliche Polysaccharide.

Von diesen sind bevorzugt:

Bisphenol A-polycarbonat, Polystyrol, Poly-p-methylstyrol, Polytrifluormethylstyrole, Polymethylmethacrylat, Celluloseacetat, Celluloseacetobutyrat, Celluloseacetopropionat, Styrol-Acrylnitrilcopolymere, Styrol-Maleinsäureanhydridcopolymere, Methylmethacrylat-Methylacrylatcopolymere, aromatische Polyester, Tetramethylbisphenol A-polycarbonat, Polyvinylacetat, Polyisobutylen, Copolymere auf Styrol und Butadien oder Isopren und Copolymere aus Acrylnitril und Butadien, deren Monomerbausteine jeweils statistisch oder blockweise angeordnet sein können und Ethylcellulose.

Selbstverständlich lassen sich auch Mischungen der oben genannten Polymere oder auch Copolymerisate mit statistischer oder blockweiser Verteilung der Monomereinheiten verwenden.

Im erfindungsgemäßen Herstellverfahren liegt das filmbildende, wasserunlösliche Polymer als Lösung in einem organischen Lösungsmittel vor, bevorzugt in einem Konzentrationsbereich zwischen 1 und 30 Gew.-%. Besonders bevorzugt ist eine Konzentration zwischen 5 und 15 Gew.-%. Das Lösungsmittel kann mit jenem der organischen Phase der Dispersion des wasserlöslichen Polymers identisch oder auch davon verschieden sein. In beiden Fällen sollen die verwendeten organischen Lösungsmittel

a) mit Wasser eine Mischungslücke aufweisen;

b) das hydrophile Polymer nicht aus der wäßrigen Phase extrahieren;

c) Siedepunkte unter 150 $^\circ$ C, vorzugsweise unter 120 $^\circ$ C, besonders bevorzugt unter 100 $^\circ$ C, aufweisen.

Geeignete Lösungsmittel für eine vorgegebene Kombination aus hydrophilen und hydrophoben Polymeren lassen sich an Hand einfacher Vorversuche ermitteln.

Verwendbar sind z.B. Ether wie Diethylether, Dipropylether oder Diisobutylether, Ketone wie Ethylmethylketon, Diethylketon oder Methylisobutylketon, Ester wie Ethylacetat, Butylacetat oder Diethylcarbonat, höhere Alkohole wie Butanol, Pentanol, Hexanol oder Cyclohexanol, aliphatische und aromatische Kohlenwasserstoffe wie Hexan, Paraffingemische, Cyclohexan, Toluol, xylol und halogenierte Kohlenwasserstoffe wie Trichlorethylen, Chloroform, Tetrachlorkohlenstoff, Perchlorethylen, Methylenchlorid, Chlorbenzol sowie auch Gemische aus den genannten oder ähnlichen Lösungsmitteln.

Bevorzugt verwendet werden aliphatische oder aromatische Kohlenwasserstoffe, die gegebenenfalls halogeniert sind, bzw. Gemische davon. Genannt seien Hexan, Cyclohexan, Paraffingemische wie ® Isopar M, Toluol, Trichlorethylen, Chloroform, Perchlorethylen und Methylenchlorid.

Die hydrophilen Polymere, die erfindungsgemäß eingesetzt werden, können natürlicher oder synthetischer Herkunft, ionisch oder nichtionisch sein. Verwendbar sind z.B. Stärke, Gelatine bzw. Derivate davon, Agar-Agar, Pullulane, hydrophile Cellulosederivate sowie hydrophile Polymerisate und Copolymerisate. Von diesen seien erwähnt: Polyethylenimin, Polyacryl- und methacrylderivate wie Polyacrylamid, Poly-N-Methyl- oder N,N-Dimethylacrylamid, Polyvinylalkohol, Polyacrylsäure und Salze davon, Polyalkylaminoalkylmethacrylate oder -amide und Salze von diesen, Polystyrolsulfonsäure und Salze davon, Polyacrylamido-2- methyl-2-propansulfonsäure bzw. Salze davon, Polyvinylimidazol, Poly-N-vinyllactame wie Poly-N-vinylpyrrolidon oder Polyvinylcaprolactam, Poly-N-vinylamide wie Poly-N-vinyl-N-methylacetamid, Poly-N-vinylurethane, Copolymere mit Maleinsäureanhydrid und Copolymere, die Grundbausteine aus obigen Polymeren statistisch oder blockweise verteilt enthalten.

Die hydrophilen Polymere werden vorzugsweise unter Zusatz eines Detergens als Emulgierhilfsmittel, das entsprechend der Kombination der hydrophilen und hydrophoben Phase aus Mc'Cutcheons Annual 1971 ausgewählt werden kann, in der organischen Phase dispergiert, für die die gleichen Kriterien gelten wie für das organische Lösungsmittel zur Herstellung der Lösung des wasserunlöslichen, filmbildenden Polymers. Beispielhaft seien ® Span-, ® Tween-, ® Tritontypen, ® Pluronics und Na-Dodecylbenzolsulfonat als mögliche Detergentien genannt. Der Gewichtsanteil der Detergentien, bezogen auf die organische Phase, liegt zwischen 0 und 50 %, vorzugsweise zwischen 1 und 30 %.

Die Menge der Detergentien sollte so gewählt werden, daß eine feine Dispersion mit einer ausreichenden Stabilität für die Dauer des Herstellungsvorgangs der Membran entsteht.

Die organische Phase bzw. das organische Lösungsmittel, in dem die hydrophilen Polymere dispergiert werden, kann in günstigen Fällen bereits das filmbildende, wasserunlösliche Polymer enthalten, so daß der Verfahrensschritt der Mischung der w/o-Dispersion des hydrophilen Polymers mit der Lösung des filmbildenden Polymers entfällt.

Die hydrophilen Polymere, die im organischen Lösungsmittel dispergiert werden, können unterschiedliche Mengen Wasser enthalten, mindestens jedoch 20 Gew.-%. Der Wassergehalt kann je nach Polymertyp wesentlich höher liegen und kann bei wasserunlöslichen, aber wasserquellbaren Typen über den ganzen Bereich bis zur maximalen Quellung bei Raumtemperatur variiert werden. Wasserlösliche Polymere werden als wäßrige Lösung verwendet in einer Konzentration, die über ein Prozent beträgt. Bevorzugt ist ein Wassergehalt der hydrophilen Polymere zwischen 50 und 5000 Gew.-%, bezogen auf das hydrophile Polymer. Besonders bevorzugt ist ein Bereich zwischen 100 und 2000 Gew.-%. Dabei ist zu beachten, daß ein Wassergehalt von beispielsweise 200 Gew.-% sowohl durch Quellung (Wasseraufnahme) eines unlöslichen Polymers als auch durch Wasseraufnahme eines wasserlöslichen Polymers erreicht werden kann, wobei man im letzteren Fall auch von einer stark konzentrierten Lösung des Polymers im Wasser spricht. Die Fähigkeit zur Wasseraufnahme ist bei unlöslichen Polymeren beschränkt durch den maximalen Quellungsgrad, der von der chemischen Natur und insbesondere der Vernetzung abhängt. Wassergehalte über 100 Gew.-% lassen sich auf alle Fälle mit wasserlöslichen Polymeren erzielen.

Zur Wasseraufnahme werden die hydrophilen Polymere mit der erforderlichen Wassermenge versetzt und, falls notwendig, mechanisch zerkleinert, um eine feine Verteilung bei der anschließenden Dispergierung im organischen Lösungsmittel zu erreichen. Bei unlöslichen Polymeren ist es von Vorteil, wenn diese schon vor der Quellung als feine Partikel vorliegen. Wäßrige Lösungen mit niedriger Viskosität lassen sich durch Zusammengießen der wäßrigen Polymerlösung und der organischen Phase und kräftiges Schütteln bereits in feiner Form verteilen. Hilfsmittel zum Verteilen wie z.B. Ultrahochgeschwindigkeitsrührwerke, Ultraschall oder spezielle Mischdüsen, die mit hohen Drücken betrieben werden, sind vor allem beim Dispergieren von viskosen Lösungen, Gelen oder gequollenen Teilchen von Vorteil. Das Volumenverhältnis der hydrophilen zur organischen Phase sollte so gewählt werden, daß der Dispergiervorgang optimal abläuft, wozu je nach Dispergierverfahren eine bestimmte Viskosität der Mischung erforderlich ist. Diese Viskosität kann durch Auflösen von organischen Polymeren im organischen Lösungsmittel erhöht werden.

Bevorzugte Volumenverhältnisse der hydrophilen zur organischen Phase liegen zwischen 1:100 und 2:1, besonders bevorzugte Verhältnisse zwischen 1:25 und 1,5:1. Ganz besonders ist ein Volumenverhältnis zwischen 1:10 und 1:1 bevorzugt.

Nach dem Dispergiervorgang kann die Dispersion wieder verdünnt oder aufkonzentriert werden, um ihre Viskosität zum Beispiel der Viskosität der Lösung des hydrophoben Polymers in einem organischen Lösungsmittel anzugleichen, mit der die Dispersion zur Membranherstellung gemischt wird. Dieser Mischvorgang kann entweder durch kräftiges Schütteln oder auch unter Zuhilfenahme mechanischer Vorrichtungen wie z.B. Rührwerke oder Mischdüsen durchgeführt werden. Das Volumenverhältnis der W/O-Dispersion der hydrophilen, wasserenthaltenden Polymere zu der Lösung des wasserunlöslichen, filmbildenden Polymers richtet sich bei vorgegebenen Konzentrationen der beiden Phasen nach dem weiter unten beschriebenen, für die Funktion der Membran erforderlichen Anteilen an hydrophilen und wasserunlöslichen, filmbildenden Polymeren. Ist aus technischen Gründen ein bestimmtes Volumenverhältnis vorgegeben, so muß das Verhältnis der Anteile an filmbildenden und hydrophilen Polymere in der Membran durch deren Konzentrationen in den zur Membranherstellung verwendeten Phasen eingestellt werden.

Das Dispergieren der hydrophilen, wasserenthaltenden Polymere im organischen Lösungsmittel kann ebenso wie das Mischen dieser Dispersion mit der Lösung des filmbildenden, wasserunlöslichen Polymers bei jeder Temperatur vorgenommen werden, die zwischen den Erstarrungs- und Siedepunkten der verwendeten Lösungsmittel liegt. Bevorzugte Temperaturen liegen zwischen 0 und $100\,^{\circ}$C. Besonders bevorzugt ist Raumtemperatur.

Zur Herstellung der Membran wird die oben beschriebene Mischung auf eine Unterlage aufgebracht, was durch Verstreichen, Aufsprühen, Auftragen mit einem Rakel oder durch Gießen erreicht werden kann. Gleichmäßige Auftragsstärken lassen sich am besten mit entsprechenden maschinellen Vorrichtungen erzielen, die dem Fachmann bekannt sind. Da die Viskosität der Mischung in weiten Grenzen variierbar ist, kann für jede Gießtechnik ein optimales Gießverhalten eingestellt werden. Die Dicke des Naßauftrags liegt vorzugsweise zwischen 10 $\mu$m und 1000 $\mu$m und richtet sich nach dem Feststoffgehalt der Mischung und der gewünschten Dicke der fertigen Membran. Diese wird durch Trocknung der auf die Unterlage aufgebrachten Mischung erhalten. Die Trocknung kann dabei durch Aufblasen von erwärmter Luft, durch Erwärmen oder durch Anlegen von Vakuum beschleunigt werden. Niedrigsiedende Lösungsmittel verdampfen u.U. auch ohne zusätzliche Maßnahmen bei Raumtemperatur genügend rasch.

Bei einer bevorzugten Ausführungsform der Trocknung wird diese mit Luft hoher relativer Feuchte durchgeführt. Geeignete Luftfeuchtigkeiten liegen zwischen 20 und 100% relative Feuchte, bevorzugt zwischen 40 und 100%. Die besten Ergebnisse werden bei Trocknung mit Luft erzielt; die 60 - 95% relative Feuchte enthält.

Die Trocknung läßt sich auch in mehreren Stufen durchführen, wobei vorteilhaft in der 1. Stufe mit Luft hoher Feuchte getrocknet wird. In den folgenden Stufen kann Luft mit niedrigeren Feuchten und z.B. höheren Temperaturen verwendet werden. Die letzte Trocknungsstufe sollt mit erwärmter Luft(Temperatur zwischen $20\,^{\circ}$ und $80\,^{\circ}$C, bevorzugt zwischen $30\,^{\circ}$ und $60\,^{\circ}$C) geringen Feuchtigkeitsgehaltes erfolgen, wenn eine vollständige Trocknung der Membran gewünscht wird.

In einer bevorzugten Ausführungsform der Erfindung wird die Dispersion des hydrophilen, wasserenthaltenden Polymers im organischen Lösungsmittel (Ölphase) durch umgekehrte Emulsionspolymerisation (Wasser-in-Öl) einer wäßrigen Lösung von Monomeren oder Monomergemischen, die in der Ölphase verteilt ist, hergestellt. Das Verfahren der umgekehrten Emulsionspolymerisation ist bekannt und beispielsweise in den US-PS 3 284 393 und 3 691 124, den DE-OS 1 745 176, 2 432 699 und 2 926 103, der EP-A 68 955 und der GB-PS 1 482 515 beschrieben. Dort sind auch Emulgatoren, polymere Stabilisatoren, Initiatoren, Ölphasen und mögliche Monomere und Monomerkombinationen, die vorteilhaft eingesetzt werden können, aufgeführt. Zur Herstellung der erfindungsgemäßen Membranen bzw. Reagenzträgerschichten besonders geeignete W/O-Dispersionen werden durch umgekehrte Emulsionspolymerisation der folgenden neutralen, basischen oder sauren Monomere oder Kombinationen davon erhalten:

Acrylamid, Methacrylamid, N,N-Dimethylacrylamid, N-Vinylpyrrolidon, N-Vinylcaprolactam, N-Vinyl-N-methylacetamid, N-Vinylacetamid, N-Vinylurethane; Acrylsäure, Methacrylsäure, Styrolsulfonsäure, Acrylamido-2-methyl-2-propansulfonsäure bzw. Salze von diesen, N-Vinylimidazol, Dimethylaminoethylmethacrylat bzw. -methacrylamid, Dimethylaminopropylmethacrylat bzw. -methacrylamid, bzw. Salze davon, sowie Mischungen aus diesen Monomeren. Ganz besonders bevorzugt als Monomere sind Acrylamid und Mischungen der genannten Monomere mit Acrylamid.

Als Initiatoren, die die Polymerisation der Monomere auslösen, sind alle Radikalbildner geeignet wie z.B. Peroxo- oder Azoverbindungen. Auch photoaktivierte Initiatorsysteme können eingesetzt werden. Vorteilhaft werden die Initiatoren aus der Gruppe der Azo-Verbindungen ausgewählt, da z.B. Reste von nicht vollständig verbrauchten peroxidischen Initiatoren mit empfindlichen Nachweisreagenzien reagieren können, die bei der Herstellung der Reagenzträgerschicht dieser zugefügt werden.

Die wäßrige Phase der W/O-Dispersion kann Zusätze enthalten wie Salze, Verdickungsmittel oder Molekulargewichtsregler. Ebenso können auch der organischen Phase der Dispersion vor oder nach der Polymerisation Substanzen zugefügt werden, die z.B. die Stabilität der Dispersion verbessern, die Viskosität erhöhen oder die Oberflächenspannung beeinflussen oder die Dispersion kann nach ihrer Herstellung verdünnt, aufkonzentriert oder teilweise entwässert werden. Vorzugsweise enthält die W/O-Dispersion polymere Stabilisatoren, die z.B. in den US-PS 3 691 124, 3 979 349 und 3 948 866 oder der GB-PS 1 482 515 aufgeführt sind.

Falls erforderlich, können unterschiedliche W/O-Dispersionen gemischt werden, um z.B. Dispersionen mit unterschiedlichen Teilchengrößen oder unterschiedliche hydrophile Polymere, die sich im Typ oder der mittleren Molmasse unterscheiden, zu kombinieren. Weiterhin können wasserlösliche Polymere nach Herstellen der W/O-Dispersion durch Umsetzung mit geeigneten Reagenzien vernetzt werden.

Bei der Herstellung der erfindungsgemäßen Membranen bzw. Reagenzträgerschichten können der Mischung aus der Lösung der filmbildenden, wasserunlöslichen organischen Polymere und der Dispersionen der hydrophilen, wasserenthaltenden Polymere verschiedene eigenschaftsverändernde Zusätze zugefügt werden. Zur Erhöhung der Reflexion können Pigmente wie Titandioxid, Kieselgel und dergleichen in der Gießmischung verteilt werden. Andere mögliche Zusätze sind Verdickungsmittel zur Erhöhung der Viskosität oder Verbindungen, die geeignet sind, die Haftung der Reagenzträgerschicht auf dem Träger zu verbessern, wenn die Reagenzträgerschicht auf dem Träger verbleibt oder zu verringern, wenn die Reagenzträgerschicht vom Träger abgelöst werden soll. Mit Hilfe von oberflächenaktiven Substanzen oder Netzmitteln lassen sich die Oberflächeneigenschaften der Reagenzträgerschicht verändern, um z.B. die Benetzbarkeit durch das Probengut zu verbessern oder die Brillianz der Reaktionsfarbe zu erhöhen. Die Eigenschaften der Reagenzträgerschicht werden auch durch Weichmacher beeinflußt, so z.B. die Härte, die Festigkeit und die Haftung.

Obwohl ein wesentlicher Vorteil der erfindungsgemäßen Reagenzträgerschicht darin besteht, daß nur eine Schicht alle Funktionen eines diagnostischen Mittels zur Bestimmung von Bestandteilen wäßriger Systeme erfüllt, lassen sich mit den erfindungsgemäßen Reagenzträgerschichten auch mehrschichtige analytische Elemente aufbauen, um z.B. unverträgliche Reaktionskomponenten räumlich zu separieren, die ansonsten bereits bei der Herstellung der Reagenzträgerschicht miteinander reagieren würden, oder um den schrittweisen Ablauf einer Reaktionsfolge zu beeinflussen, indem für jede Reaktionsstufe optimale Bedingungen (z.B. unterschiedliche pH-Werte) in verschiedenen Reagenzträgerschichten geschaffen werden. Eine 2. Reagenzträgerschicht, die der eigentlichen Reagenzträgerschicht, die die Nachweisreagenzien für eine bestimmte Substanz enthält, überlagert ist, kann auch die Aufgabe erfüllen, Verbindungen, die die Nachweisreaktion stören, zu entfernen, d.h. nicht hindurchtreten zu lassen oder unschädlich zu machen. Dies kann durch physikalische oder chemische Methoden erreicht werden. Eine oder mehrere reagenzfreie Abdeckschichten können als Filter-, Spreit- oder Reflexionsschichten dienen. Zur Erzielung hoher Schichtdicken können mehrere Reagenzträgerschichten, die die gleichen Reagenzien enthalten, übereinander gegossen werden.

Die erfindungsgemäßen Reagenzträgerschichten lassen sich auch mit an sich bekannten Schichten kombinieren, die für analytische Testmittel verwendet werden, wie z.B. Gelatine oder Polyvinylalkohol. Bei einer Kombination mit hydrophilen Schichten lassen sich besonders gut Reagenzien voneinander getrennt halten, da vor allem während des Herstellungsvorgangs der Multischichten durch aufeinanderfolgende Gießvorgänge keine Vermischung der hydrophilen und hydrophoben Gießmischungen bzw. Schichten erfolgt.

Zur Ausführung einer Analyse unter Verwendung der erfindungsgemäßen Reagenzträgerschichten wird ein Teststreifen, der mindestens eine Reagenzträgerschicht mit geeigneten Nachweisreagenzien, z.B. für eine Glucosebestimmung, sowie eine Trägerfolie aufweist, in die zu untersuchende Flüssigkeit, z.B. Urin eingetaucht, herausgezogen und die gebildete Färbung visuell oder spektroskopisch ausgewertet. Besteht die zu untersuchende Flüssigkeit aus Vollblut, wird ein Tropfen davon auf eine Reagenzträgerschicht aufgebracht, kurze Zeit gewartet und überschüssige Flüssigkeit einschließlich der Erythrocyten einfach abgewischt. Die gebildete Färbung kann dann ausgewertet werden. Die Tropfengröße hat dabei keinen Einfluß auf die Farbintensität, so daß das Probevolumen nicht exakt dosiert werden muß.

Der Anteil der Flüssigkeitsprobe, der von der Reagenzträgerschicht aufgenommen wird, hängt außer von der Natur der hydrophoben, filmbildenden und der hydrophilen Polymere und evtl. Zusätzen, z.B. zur Oberflächenmodifizierung, auch von dem Verhältnis der Anteile der hydrophoben, filmbildenden und der hydrophilen Polymere ab. Dieses Verhältnis beeinflußt auch die Wischfestigkeit der Reagenzträgerschicht. Während bei sehr großen Anteilen an hydrophilen Polymeren nicht wischfeste, stark wasseraufnehmende Schichten entstehen, führen sehr große Anteile an hydrophoben, filmbildenden Polymeren zu sehr festen Filmen, die jedoch nur noch geringe Mengen an wäßriger Flüssigkeit aufnehmen. Je nach Verwendungszweck können somit die Eigenschaften der Reagenzträgerschicht eingestellt werden. Für die Analyse von Vollblut muß die Reagenzträgerschicht z.B. wischfest sein, um die Erythrocyten entfernen zu können, während für eine Urinanalyse Wischfestigkeit nicht erforderlich ist. Für letztere kann eine größere Hydrophilie der Reagenzträgerschicht von Vorteil sein, damit bei kurzem Eintauchen des Teststreifens in die Urinprobe eine genügend große Flüssigkeitsmenge festgehalten wird. Vorteilhaft liegt das Gewichtsverhältnis der Anteile hydrophober, filmbildender zu hydrophiler, wasserenthaltender Polymere in den Reagenzträgerschichten zwischen 1:10 und 10:1. Bevorzugt liegt dieses Verhältnis zwischen 1:5 und 5:1. Ganz besonders bevorzugt ist ein Verhältnis zwischen 1:2 und 2:1.

Die folgenden Beispiele dienen zur näheren Erläuterung der vorliegenden Erfindung.

In den Beispielen 1-16 wird die Herstellung von W/O-Dispersionen wasserlöslicher Polymere durch Dispersionspolymerisation beschrieben. Solche Dispersionen werden bevorzugt zur Herstellung der erfindungsgemäßen Reagenzträgerschichten verwendet.

Beispiel 1

In einem 1 l Planschliffgefäß mit einem Blattrührer werden 440 g Chloroform, 12 g ® Span 80 (® Span 80 ist ein Sorbitanmonooleat), 4 g eines Copolymeren aus Stearylmethacrylat und Acrylamid (82,6/17,4 Gew.-Anteile) und 0,2 g Azo-bis-(isobutyronitril) vorgelegt, entgast und auf 50°C erwärmt. Bei dieser Temperatur werden unter leichtem Stickstoffstrom 20 % einer entgasten Lösung aus 40 g Acrylamid und 80 g H₂O schnell zugetropft. Der Rest der Monomerlösung wird im Verlauf von 2 h zugetropft. Nach beendeter Zugabe wird 2 h nachgerührt. Die Dispersion enthält 6,9 Gew.-% Polyacrylamid.

Beispiel 2-16

Analog Beispiel 1 werden W/O-Dispersionen hergestellt, deren Bestandteile bzw. Reaktionsbedingungen, die sich von den Angaben in Beispiel 1 unterscheiden, in Tabelle 1 aufgelistet sind.

## Tabelle 1

| Beispiel Nr. | organische Phase | Emulgator (Gewichtsanteil/%[+]) | Monomer (Gewichtsanteil/%) | H₂O-Gehalt [++] | T °C |
|---|---|---|---|---|---|
| 2 | Trichlorethylen | Span 80 (2,6) | Acrylamid (6,9) | 66,7 | 60 |
| 3 | " | Span 60 (2,6) | Acrylamid (6,9) | 60 | 70 |
| 4 | Toluol | Span 80 (4,3) | Acrylamid (8,2) | 66,7 | 55 |
| 5[+++] | Cyclohexan | Span 60 (10,0) | Acrylamid (11,2) | 60 | 80 |
| 6 | Chloroform | Span 80 (3,5) | Acrylamid (6,9) | 80 | 50 |
| 7[+++] | " | Span 80 (3,5) | " | 66,7 | 55 |
| 8 | " | Span 80 (2,6) | Acrylamid (13,2) | 66,7 | 55 |
| 9[++++] | " | Span 80 (2,6) | Acrylamid (6,9) | 66,7 | 55 |
| 10 | " | " | N,N-Dimethylacrylamid (6,9) | " | " |
| 11 | " | " | N,N-Dimethylamidnoethylmeth-acrylat·HCl/Acrylamid (1,7/5,2) | " | " |
| 12 | " | " | Na-Acrylat/Acrylamid (4,0/3,4) | " | " |
| 13 | " | " | N-Vinylpyrrolidon/Acrylamid (3,4/3,4) | " | " |
| 14 | " | " | Methacrylamid (6,9) | " | " |
| 15 | " | " | N-Vinylacetamid/Acrylamid (3,5/3,5) | " | " |
| 16 | " | " | N-Vinylimidazol/Acrylamid (3,5/3,5) | " | " |

+ bezogen auf organische Phase
++ bezogen auf wäßrige Phase
+++ ohne Stearylmethacrylat-Acrylamid-Copolymer-Zusatz
++++ mit 0,5 g Azo-bis(isobutyronitril)

Beispiel 17 beschreibt die einfache Herstellung einer erfindungsgemäßen Reagenzträgerschicht, die Nachweisreagenzien für eine Glucosebestimmung enthält.

## Beispiel 17

6 mg Glucoseoxidase (150 U/mg) und
6 mg Peroxidase (80 U/mg) werden in
0,2 ml 1 m Citratpufferlösung (pH 5,5) gelöst.

Diese Lösung wird in
0,4 ml Chloroform, das
40 mg Dodecylbenzolsulfonat enthält, durch Schütteln in Form feiner Tröpfchen verteilt. Die entstandene Dispersion wird zu einer Mischung aus
2,5 ml Dispersion aus Beispiel 1,
2,5 ml einer 7,5 %igen Lösung von Polycarbonat (hergestellt durch kontinuierliche Phasengrenzflächenpolykondensation; $\eta_{rel}$ = 1,30, gemessen in 0,5 % iger Lösung in Methylenchlorid) in Chloroform und 0,1 g Tetramethylbenzidin
gegeben. Der Ansatz wird kräftig geschüttelt und mit einem Rakel auf eine Polyesterfolie aufgebracht (Naßauftrag 250 $\mu$m). Nach kurzer Trockenzeit bei Raumtemperatur wird der fertige Reagenzfilm erhalten.

Zur Bestimmung der Glucosekonzentration von Vollblut wird ein Tropfen Blut auf den Reagenzfilm aufgebracht und nach 15 sec. abgewischt. Nach 1 min ist die Nachweisreaktion beendet und die Glucosekonzentration der Blutprobe kann anhand der Farbintensität der Testzone bestimmt werden. Für Glucosekonzentrationen im Bereich zwischen 0 und 500 mg/dl werden proportional zur Konzentration der Probelösung zunehmende Farbintensitäten der Testzone beobachtet. Die Farbintensität ist dabei nicht vom Volumen der aufgebrachten Probe abhängig. Bei Verwendung transparenter Polyesterfolien als Unterlage kann die Färbung sowohl von oben als auch von unten beobachtet werden.

Zur Bestimmung von Glucose in Urin wird der trockene Reagenzfilm in schmale Streifen geschnitten, die jeweils kurze Zeit in Urinproben unterschiedlichen Glucosegehaltes getaucht werden. Die Farbreaktion ist nach etwa 1 min. beendet. In Abhängigkeit von der Glucosekonzentration werden hinsichtlich ihrer Intensität gut abgestufte Blaufärbungen im Bereich zwischen 0 und 500 mg/dl Glucose erhalten.

In analoger Weise wie für den Nachweis von Analyten in Vollblut und Urin gezeigt lassen sich auch die Inhaltsstoffe anderer wäßriger Flüssigkeiten bestimmen.

In den folgenden Beispielen werden analog Beispiel 17 Glucosetestfilme unter Verwendung der in Tab. 2 aufgelisteten Dispersionen und hydrophoben Polymere hergestellt.

**Tabelle 2**

| Beispiel-Nr. | Dispersion aus Beispiel Nr. (ml) | Polymer | Lösungsmittel | Volumen der Polymerlösung (ml) |
|---|---|---|---|---|
| 18 | 6 (2,5) | 1 | $CHCl_3$ | 2,5 |
| 19 | 7 (2,5) | 2 | $CHCl_3$ | 2,5 |
| 20 | 9 (2,5) | 3 | $CHCl_3$ | 2,5 |
| 21 | 9 (2,5) | 4 | $CHCl_3$ | 2,5 |
| 22 | 6 (2,5) | 5 | $CHCl_3$ | 2,5 |
| 23 | 8 (1,3) | 6 | $CHCl_3$ | 3,7[+] |
| 24 | 8 (2) | 7 | $CHCl_3$ | 3 |
| 25 | 8 (2) | 8 | $CHCl_3$ | 3 |
| 26 | 8 (2) | 9 | $CH_2Cl_2$ | 3 |
| 27 | 5 (2,5) | 10 | Cyclohexan | 2,5 |
| 28 | 9 (2,5) | 11 | $CHCl_3$ | 2,5 |
| 29 | 4 (2) | 12 | Toluol | 3 |
| 30 | 8 (1,7) | 12 | $CHCl_3$ | 3,3 |
| 40 | 9 (2,5) | 13 | $CHCl_3$ | 2,5 |
| 41 | 2 (2,5) | 14 | $CHCl_3$ | 2,5 |
| 42 | 3 (2,5) | 15 | $CHCl_3$ | 2,5 |
| 43 | 13 (2,5) | 16 | $CHCl_3$ | 2,5 |

Tabelle 2 (Fortsetzung)

| Beispiel-Nr. | Dispersion aus Beispiel Nr. (ml) | Polymer | Lösungsmittel | Volumen der Polymerlösung (ml) |
|---|---|---|---|---|
| 44 | 10 (2,5) | 17 | $CHCl_3$ | 2,5 |
| 45 | 8 (2) | 18 | $CHCl_3$ | 3 |
| 46 | 11 (2,5) | 17 | $CHCl_3$ | 2,5 |
| 47 | 12 (2,5) | 17 | $CHCl_3$ | 2,5 |
| 48 | 14 (2,5) | 17 | $CHCl_3$ | 2,5 |
| 49 | 15 (2,5) | 17 | $CHCl_3$ | 2,5 |
| 50 | 16 (2,5) | 17 | $CHCl_3$ | 2,5 |
| 51 | 6 (2,5) | 19 | $CHCl_3$ | 2,5 |

+ 5 %ige Lösung

Chemische Zusammensetzung der verwendeten hydrophoben Polymere:

1 Copolymer aus 80 % Styrol und 20 % Acrylnitril;

2 Polymethylmethacrylat; $[\eta]$ (25° C, THF) = 0,6

3 Copolymer aus 80 % Styrol und 20 % Maleinsäureanhydrid;

4 Copolymer aus 75 % Styrol, 15 % Acrylnitril und 10 % Maleinsäureanhydrid;

5 Copolymer aus 56 % Styrol, 28 % Methylmethacrylat und 16 % Maleinsäureanhydrid;

6 Celluloseacetat; $\eta_{rel}$ (20 %ig in Aceton:Ethanol = 9:1) = 180

7 Celluloseacetobutyrat; $\eta_{rel}$ (20 %ig in Aceton:Ethanol = 9:1) = 200

8 Celluloseacetopropionat; $\eta_{rel}$ (20 %ig in Aceton:Ethanol = 9:1) = 200

9 Polycarbonat aus Tetramethylbisphenol A; $\eta_{rel}$ (gemessen 0,5 %ig in Methylenchlorid) = 1,33

10 Polyisobutylen; (® Oppanol B 100)

11 Polyester aus Tetramethyl-bisphenol A und Terephthalsäure; $\eta_{rel}$ (gemessen 0,5 %ig in Methylenchlorid) = 1,4

12 Polystyrol; $[\eta]$ = 0,9 (Toluol, 25° C)

13 Polyvinylacetat; Molekulargewicht: 37.000 g/Mol

14 Polycarbonat aus Bisphenol A; $\eta_{rel}$, gemessen 0,5 %ig in Methylenchlorid, = 1,28

15 Polycarbonat aus Bisphenol A; $\eta_{rel}$ (0,5 %ig in Methylenchlorid) = 1,29

16 Polycarbonat aus Bisphenol A, mit 0,5 Mol-% Isatinbiskresol als Verzweiger; $\eta_{rel}$ (0,5 %ig in Methylenchlorid) = 1,32

17 Polycarbonat aus Bisphenol A; $\eta_{rel}$ gemessen 0,5 %ig in Methylenchlorid, = 1,30

18 Poly-p-Methylstyrol; $[\eta]$ = 0,8 (Toluol, 25° C)

19 SBS-3-Block-Copolymer aus 30 % Styrol und 70 % Butadien, hergestellt durch anionische Polymerisation in Hexan mit Butyllithium als Initiator, Molekulargewicht: 150000 g/Mol

Die Glucosetestfilme aus den Beispielen 18-51 zeigen nach Aufbringen von Testlösungen jeweils eine gute Abwischbarkeit und proportional zur Konzentration der Testlösungen an Glucose zunehmende Farbintensitäten.

Das folgende Beispiel 52 verdeutlicht den Einfluß des Verhältnisses der Anteile an hydrophilem zu hydrophobem Polymer auf die Eigenschaften der erfindungsgemäßen Reagenzträgerschicht.

Beispiel 52

Mit der Dispersion aus Beispiel 8 werden gemäß Beispiel 17 Glucosetestfilme hergestellt, wobei durch Variation der Anteile an Dispersion und Polycarbonatlösung verschiedene Gewichtsverhältnisse von Polyacrylamid zu Polycarbonat eingestellt werden.

| Gewichtsverhältnis Polyacrylamid/Polycarbonat | Filmeigenschaften |
|---|---|
| 5:1 | ungleichmäßiger Film, zerfließt mit Wasser |
| 2:1 1:1 1:2 | glatte Filme, gute Wischfestigkeit, gleichmäßige Färbung; von 2:1 nach 1:2 nimmt die Wasseraufnahme ab und die Wischfestigkeit zu. |
| 1:5 | Film färbt unregelmäßig; Farbe wird beim Abwischen der Probelösung entfernt. |

Beispiel 53 zeigt die Eignung der erfindungsgemäßen Reagenzträgerschichten für den Nachweis hochmolekularer Verbindungen, indem ein Testfilm für Glucoseoxidase hergestellt wird.

Beispiel 53

15 mg Peroxidase (80 U/mg) und
50 mg Glucose werden in

0,5 ml Citratpufferlösung (1m, pH 5,5) gelöst.

Diese Lösung wird in einer Lösung aus

0,1 g Dodecylbenzolsulfonatpaste (75 %ig),

0,1 g Tetramethylbenzidin und

0,1 g Polymer 15 (aus Beispiel 42) in

1,0 ml Chloroform

dispergiert.

Von dieser Dispersion werden 0,2 ml zu einer Mischung aus 2,5 ml Dispersion aus Beispiel 1 und 2,5 ml einer 7,5 % gen Lösung von Polymer 15 in Chloroform gegeben und gut vermischt. Mit der Mischung wird mittels eines Rakels ein Film auf Polyesterfolie gezogen (Naßauftrag 250 $\mu$m) und bei Raumtemperatur getrocknet.

Nach Aufbringen von Glucoseoxidase-Lösungen unterschiedlicher Konzentrationen auf den Reagenzfilm und Abwischen der Lösungen nach 1 min, lassen sich nach 1 weiterer Minute den Konzentrationen entsprechende Werte für die Reflexionsintensitäten der Testzone in einem Reflexionsphotometer quantitativ bestimmen.

In den Beispielen 54-55 wird die Eignung der erfindungsgemäßen Reagenzträgerschichten für den Nachweis weiterer Analyten gezeigt.

Beispiel 54

Ein Testfilm für Kaliumionen wird hergestellt, indem 2,5 ml Dispersion aus Beispiel 1 und

0,2 ml 2-Nitrophenylbutylether

mit einer Lösung aus

3,5 mg Gibbs-Reagenz (7-(n-decyl)-2-methyl-4-(3',5'-dichlorchinon-4-imido)1-naphthol),

9,0 mg Kronenether (2,3 Naphtho-15-Krone-5) und 2,5 ml einer 7,5 %igen Lösung von Polymer 17 in Chloroform vermischt und auf eine Polyesterfolie in einer Schichtdicke von 200 $\mu$m aufgetragen werden. Der getrocknete Film ergibt mit Kaliumchloridlösung unterschiedlicher Konzentrationen (zwischen 0 und 9 mMol/l) unterschiedlich starke Blaufärbungen.

Beispiel 55

Zur Herstellung eines Reagenzfilms für den Nachweis von Acetoacetat wird eine Lösung aus

12,3 g Mg SO$_4$ und

1,3 g Na$_2$Fe (CN)$_5$NO.2H$_2$O in

15 ml Wasser

mittels 45 %iger Natronlauge auf pH 9-10 eingestellt.

Von dieser Lösung werden 12 ml in 32 ml einer 7,5 %igen Lösung von Polymer 17 in Chloroform, die 2,3 g Dodecylbenzolsulfonatpaste (75 %ig) enthält, dispergiert. Von dieser Dispersion werden 2,5 ml mit 2,5 ml Dispersion aus Beispiel 1 gemischt und mittels eines Rakels auf Polyethylen-beschichtetes Papier aufgetragen (250 $\mu$m).

Nach dem Trocknen bei Raumtemperatur werden beim Testen mit Acetoacetatlösungen entsprechend deren Konzentrationen in ihrer Intensität gut abgestufte Rotfärbungen erhalten.

Beispiel 56

Herstellen eines Glucoseteststreifens:

60 ml einer Lösung von Celluloseacetobutyrat (Polymer 7 in Tab. 2) in Chloroform (7,5-Gew.%), die 1 g Tetramethylbenzidin und 1,2 g Natriumdodecylbenzolsulfosat enthält, werden mit

30 ml einer 6,8 %igen Lösung von Polyvinylpyrrolidon (Molekulargewicht 400.000 g/Mol) in 0,1 m Citratpuffer (pH 5,5)

kräftig geschüttelt und mit einem Druck von 9 bar durch eine Düse ($\varnothing$ der Bohrung 0,3 mm) gedrückt. Dabei entsteht eine feinteilige Dispersion.

50 ml dieser Dispersion werden mit einer Dispersion aus einer Lösung von

11 mg Glucoseoxidase (200 U/mg)

60 mg Peroxidase (80 U/mg) in

2 ml Citratpuffer (1 m, pH 5,5)

dispergiert in

4 ml Chloroform, das

0,4 g Natriumdodecylbenzolsulfonat enthält,
gemischt und durch einer Schlitzgießer (1,8 cm breit) auf Polyesterfolie gegossen (180 μm Naßauftrag). Die Trocknung erfolgt durch Beblasen mit Luft:
10 min mit Luft von 25° C und 90% rel. Feuchte und anschließend 10 min mit Luft von 40° C und 20% rel. Feuchte

Der fertige Testfilm wird auf eine weiße Trägerfolie geklebt und in 0,5 cm breite Streifen geschnitten. Die Streifen werden mit Vollblutproben mit Glucosegehalten zwischen 20 und 400 mg/dl getestet (Einwirkzeit bis zum Abwischen 15 sec). Nach jeweils 1 Minute ist die Nachweisreaktion beendet. Die Farbintensität des Testfeldes nimmt mit steigender Glucosekonzentration der Blutproben gleichmäßig zu.

**Ansprüche**

1. Wasser aufnehmende Membran für Reagenzträgerschichten in analytischen Mitteln zum trockenchemischen Nachweis von Bestandteilen wäßriger Probelösungen, dadurch gekennzeichnet, daß die Membran ein oder mehrere hydrophobe filmbildende, wasserunlösliche organische Polymere aus der Gruppe Polykohlenwasserstoffe, Polydiene, Polyvinylverbindungen, Polyacrylate bzw. -methacrylate, Polycarbonate, Polyacetale, Polyether, Polyurethane, Phenolharze, Polyester, Polysulfone, Polyamide bzw. -imide und organolösliche Polysaccharide enthält, in welchen ein oder mehrere hydrophile organische Polymere, die mindestens 20 Gewichtsprozent Wasser aufnehmen als diskrete Partikel verteilt sind.

2. Membran nach Anspruch 1, dadurch gekennzeichnet, daß die hydrophilen Polymere in den Poren eingelagert sind, wobei die kohärente Phase der Membran aus den hydrophoben, wasserunlöslichen Polymeren besteht.

3. Membran nach Anspruch 1 oder 2, dadurch gekennzeichnet, daß das Gewichtsverhältnis von hydrophilen zu hydrophoben Polymeren zwischen 10:1 und 1:10, bevorzugt zwischen 5:1 und 1:5 liegt.

4. Membran nach Anspruch 1 bis 3, dadurch gekennzeichnet, daß sie erhältlich ist, indem man die Lösung eines oder mehrerer filmbildender, hydrophober, wasserunlöslicher organischer Polymere mit einer Dispersion eines oder mehrerer hydrophiler, Wasser enthaltender organischer Polymere in einem organischen Lösungsmittel mischt, die Mischung auf eine Unterlage aufbringt und trocknet.

5. Membran nach Anspruch 1 bis 3, dadurch gekennzeichnet, daß sie erhältlich ist, indem man ein oder mehrere hydrophile, wasserenthaltende, organische Polymere in der Lösung eines oder mehrerer filmbildender, hydrophober, wasserunlöslicher organischer Polymere dispergiert, diese Dispersion auf eine Unterlage aufbringt und trocknet.

6. Reagenzträgerschicht für analytische Mittel zum trockenchemischen Nachweis von Bestandteilen wäßriger Probelösungen, dadurch gekennzeichnet, daß sie aus einer Membran nach Anspruch 1 bis 5 besteht, welche eines oder mehrere Nachweisreagenzien für die Bestimmung des nachzuweisenden Bestandteils enthält.

7. Analytisches Mittel zum trockenchemischen Nachweis von Bestandteilen wäßriger Probelösungen, dadurch gekennzeichnet, daß es mindestens eine Trägerschicht gemäß Anspruch 6 aufweist.

8. Verfahren zur Herstellung von Membranen gemäß Anspruch 1 bis 4, dadurch gekennzeichnet, daß man die Lösung eines oder mehrerer filmbildender, wasserunlöslicher organischer Polymere mit einer Dispersion eines oder mehrerer hydrophiler mindestens 20 Gew.-% Wasser enthaltender organischer Polymere in einem organischen Lösungsmittel mischt, die Mischung auf eine Unterlage aufbringt und trocknet.

9. Verfahren zur Herstellung von Membranen gemäß Anspruch 1 bis 3 und 5, dadurch gekennzeichnet, daß man ein oder mehrere hydrophile, wasserenthaltende organische Polymere in der Lösung eines oder mehrerer filmbildender, hydrophober, wasserunlöslicher organischer Polymere dispergiert, diese Dispersion auf eine Unterlage aufbringt und trocknet.

10. Verfahren zur Herstellung einer Reagenzträgerschicht gemäß Anspruch 6, dadurch gekennzeichnet, daß

16

man im Verfahren von Anspruch 8 hydrophobe Nachweisreagenzien als Lösung in einem organischen Lösungsmittel und/oder hydrophile Nachweisreagentien in Form einer Wasser-in-Öl-Dispersion, deren wäßrige Phase die hydrophilen Nachweisreagentien gelöst enthält, der Mischung vor dem Aufbringen auf eine Unterlage zufügt.

11. Verfahren zur Herstellung einer Reagenzträgerschicht gemäß Anspruch 6, dadurch gekennzeichnet, daß hydrophobe Nachweisreagenzien in den organischen Lösungen, hydrophile Nachweisreagenzien in den wäßrigen Lösungen, die jeweils zur Herstellung der Membran nach Anspruch 4 und 5 verwendet werden, gelöst sind.

12. Verfahren zur Herstellung von Membranen gemäß Anspruch 8, dadurch gekennzeichnet, daß die Wasser-in-Öl-Dispersion des hydrophilen, wasserenthaltenden Polymers durch umgekehrte Emulsionspolymerisation von wasserlöslichen Monomeren hergestellt wird.

13. Verfahren zur Herstellung einer Reagenzträgerschicht gemäß Anspruch 6, dadurch gekennzeichnet, daß man eine Membran gemäß Anspruch 1 bis 5 mit einer oder mehreren Lösungen von Nachweisreagenzien tränkt und danach trocknet.

14. Verfahren zum trockenchemischen Nachweis von Bestandteilen einer wäßrigen Probelösung, dadurch gekennzeichnet, daß man die Probe mit einem analytischen Mittel gemäß Anspruch 6 in Kontakt bringt und die auftretende Nachweisreaktion verfolgt.

**Claims**

1. Water-absorbing membrane for reagent substrates in analytical agents for the dry-chemical detection of components of aqueous sample solutions, characterised in that the membrane comprises one or more hydrophobic film-forming, water-insoluble organic polymers from the group comprising polyhydrocarbons, polydienes, polyvinyl compounds, polyacrylates and polymethacrylates, polycarbonates, polyacetals, polyethers, polyurethanes, phenol resins, polyesters, polysulphones, polyamides, polyimides and organosoluble polysaccharides, in which one or more hydrophilic organic polymers which absorb at least 20 per cent by weight of water are dispersed as discrete particles.

2. Membrane according to Claim 1, characterised in that the hydrophilic polymers are contained in the pores, the coherent phase of the membrane consisting of the hydrophobic, water-insoluble polymers.

3. Membrane according to Claim 1 or 2, characterised in that the weight ratio of hydrophilic polymers to hydrophobic polymers is between 10:1 and 1:10, preferably between 5:1 and 1:5.

4. Membrane according to Claims 1 to 3, characterised in that it can be obtained by mixing a solution of one or more film-forming, hydrophobic, water-insoluble organic polymers with a dispersion of one or more hydrophilic, water-containing organic polymers in an organic solvent, and applying the mixture onto a base and drying it.

5. Membrane according to Claims 1 to 3, characterised in that it can be obtained by dispersing one or more hydrophilic, water-containing, organic polymers in a solution of one or more film-forming, hydrophobic, water-insoluble organic polymers, and applying this dispersion onto a base and drying it.

6. Reagent substrate for analytical agents for the dry-chemical detection of components of aqueous sample solutions, characterised in that it consists of a membrane according to Claims 1 to 5, which contains one or more detection reagents for the determination of the component to be detected.

7. Analytical agent for the dry-chemical detection of components of aqueous sample solutions, characterised in that it possesses at least one substrate according to Claim 6.

8. Process for the preparation of membranes according to Claims 1 to 4, characterised in that a solution of one or more film-forming, water-insoluble organic polymers is mixed with a dispersion, in an organic solvent, of one or more hydrophilic organic polymers which contain at least 20% by weight of water, and the mixture is applied onto a base and dried.

9. Process for the preparation of membranes according to Claims 1 to 3 and 5, characterised in that one or more hydrophilic, water-containing organic polymers are dispersed in a solution of one or more film-forming, hydrophobic, water-insoluble organic polymers, and this dispersion is applied onto a base and dried.

10. Process for the preparation of a reagent substrate according to Claim 6, characterised in that , in the process of Claim 8, hydrophobic detection reagents in the form of a solution in an organic solvent and/or hydrophilic detection reagents in the form of a water-in-oil dispersion, the aqueous phase of which contains the hydrophilic detection reagents in solution, are added to the mixture before application to a base.

11. Process for the preparation of a reagent substrate according to Claim 6, characterised in that hydrophobic detection reagents are dissolved in the organic solutions, and hydrophilic detection reagents are dissolved in the aqueous solutions, each of which is used for the preparation of the membrane according to Claim 4 and 5.

12. Process for the preparation of membranes according to Claim 8, characterised in that the water-in-oil dispersion of the hydrophilic, water-containing polymer is prepared by inverse emulsion polymerisation of water-soluble monomers.

13. Process for the preparation of a reagent substrate according to Claim 6, characterised in that a membrane according to Claims 1 to 5 is impregnated with one or more solutions of detection reagents and then dried.

14. Process for the dry-chemical detection of components of an aqueous sample solution, characterised in that the sample is brought into contact with an analytical agent according to Claim 6, and the resulting detection reaction is monitored.

## Revendications

1. Membrane absorbant l'eau destinée à des couches de support pour réactifs dans des agents analytiques destinés à la détection chimique à sec de constituants de solutions aqueuses de prélèvement, **caractérisée en ce que** la membrane contient un ou plusieurs polymères organiques insolubles dans l'eau, hydrophobes et filmogènes, choisis parmi le groupe comprenant des polyhydrocarbures, des polydiènes, des composés polyvinyliques, des polyacrylates ou polyméthacrylates, des polycarbonates, des polyacétals, des polyéthers, des polyuréthannes, des résines phénoliques, des polyesters, des polysulfones, des polyamides ou polyimides, ainsi que des polysaccherides organo-solubles, dans lesquels est ou sont réparti(s), sous forme de particules discrètes, un ou plusieurs polymères organiques hydrophiles qui absorbent au moins 20 % en poids d'eau.

2. Membrane selon la revendication 1, **caractérisée en ce que** les polymères hydrophiles sont incorporés dans les pores, la phase cohérente de la membrane étant constituée par les polymères hydrophobes et insolubles dans l'eau.

3. Membrane selon la revendication 1 ou 2, **caractérisée en ce que** le rapport pondéral des polymères hydrophiles aux polymères hydrophobes se situe entre 10:1 et 1:10, de préférence, entre 5:1 et 1:5.

4. Membrane selon les revendications 1 à 3, **caractérisée en ce qu'**on peut l'obtenir en mélangeant la solution d'un ou de plusieurs polymères organiques insolubles dans l'eau, hydrophobes et filmogènes, avec une dispersion d'un ou de plusieurs polymères organiques, hydrophiles contenant de l'eau, dans un solvant organique, en appliquant le mélange sur un substrat et en le séchant.

5. Membrane selon les revendications 1 à 3, **caractérisée en ce qu'**on l'obtient en dispersant un ou plusieurs polymères organiques, hydrophiles contenant de l'eau, dans la solution d'un ou de plusieurs polymères organiques insolubles dans l'eau, hydrophobes et filmogènes, en appliquant cette dispersion sur une substrat et en la séchant.

6. Couche de support pour réactif pour des agents analytiques destinés à la détection chimique à sec de constituants de solutions aqueuses de prélèvement, **caractérisée en ce qu'**elle est constituée d'une membrane selon les revendications 1 à 5, cette couche contenant un ou plusieurs réactifs de détection pour la détermination du constituant à détecter.

7. Agent analytique destiné à la détection chimique à sec de constituants de solutions aqueuses de prélèvement, **caractérisé en ce qu'**il présente au moins une couche de support selon la revendication 6.

8. Procédé pour la préparation de membranes selon les revendications 1 à 4, **caractérisé en ce qu'** on mélange la solution d'un ou de plusieurs polymères organiques insolubles dans l'eau et filmogènes, avec une dispersion d'un ou de plusieurs polymères organiques hydrophiles contenant au moins 20 % en poids d'eau, dans un solvant organique, on applique le mélange sur un substrat et on le sèche.

9. Procédé pour la préparation de membranes selon les revendications 1 à 3 et 5, **caractérisé en ce qu'**on disperse un ou plusieurs polymères organiques hydrophiles contenant de l'eau, dans la solution d'un ou de plusieurs polymères organiques insolubles dans l'eau, hydrophobes et filmogènes, on applique cette dispersion sur un substrat et on la sèche.

10. Procédé pour la préparation d'une couche de support pour réactif selon la revendication 6, **caractérisé en ce que**, dans le procédé de la revendication 8, on ajoute au mélange des réactifs hydrophobes d'identification sous forme d'une solution dans un solvant organique, et/ou des réactifs hydrophiles de détection sous forme d'une dispersion de type eau-dans-l'huile, dont la phase aqueuse contient les réactifs hydrophiles de détection sous forme dissoute, avant l'application sur un substrat.

11. Procédé pour la préparation d'une couche de support pour réactif selon la revendication 6, **caractérisé en ca que** les réactifs hydrophobes de détection au sein des solutions organiques, les réactifs hydrophiles de détection dans les solutions aqueuses, que l'on utilise chaque fois pour la préparation de la membrane selon les revendications 4 et 5, sont dissous.

12. Procédé pour la préparation de membranes selon la revendication 8, **caractérisé en ce que** l'on prépare la dispersion eau-dans-l'huile du polymère hydrophile contenant de l'eau, par polymérisation inverse en émulsion de monomères hydrosolubles.

13. Procédé pour la préparation d'une couche de support pour réactif selon la revendication 6, **caractérisé en ce qu'**on imprègne une membrane selon les revendications 1 à 5, avec une ou plusieurs solutions des réactifs de détection et ensuite, on la sèche.

14. Procédé pour la détection chimique à sec de constituants d'une solution aqueuse de prélèvement, **caractérisé en ce qu'**on met le prélèvement en contact avec un agent analytique selon la revendication 6 et on suit la réaction de détection qui apparaît.